(19) Europäisches Patentamt
European Patent Office
Office européen des brevets

(11) Publication number: **0 011 111**
**B1**

(12) # EUROPEAN PATENT SPECIFICATION

(45) Date of publication of patent specification: **22.09.82**

(21) Application number: **79103659.3**

(22) Date of filing: **26.09.79**

(51) Int. Cl.³: **C 07 D 513/04,**
A 61 K 31/425
//(C07D513/04, 277/00,
235/00)

(54) **5-(4-Pyridyl)-6-(4-fluorophenyl)-2,3-dihydroimidazo-(2,1-b)-thiazole, process for its preparation and medical composition.**

(30) Priority: **27.09.78 US 946260**

(43) Date of publication of application:
**28.05.80 Bulletin 80/11**

(45) Publication of the grant of the patent:
**22.09.82 Bulletin 82/38**

(84) Designated Contracting States:
**AT BE CH DE FR GB IT LU NL SE**

(56) References cited:
EP - A - 0 000 353
DE - A - 2 709 639
DE - A - 2 742 725

The file contains technical information submitted after the application was filed and not included in this specification

(73) Proprietor: **SMITHKLINE BECKMAN CORPORATION**
**1 Franklin Plaza**
**Philadelphia Pennsylvania 19101 (US)**

(72) Inventor: **Bender, Paul Elliot**
**504 Lilac Lane**
**Cherry Hill, New Jersey 08003 (US)**
Inventor: **Lantos, Ivan**
**1447 Boxwood Drive**
**Blackwood, New Jersey 08012 (US)**

(74) Representative: **Vossius Vossius Tauchner Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 011 111 B1

# 5-(4-Pyridyl)-6-(4-fluorophenyl)-2,3-dihydroimidazo-[2,1-b]-thiazole, Process for its Preparation and Medical Composition

This invention relates to a new chemical compound, namely 5-(4-pyridyl)-6-(4-fluorophenyl)-2,3-dihydroimidazo[2,1-b]-thiazole (compound I), together with its acid addition salts and S- oxide derivatives, which have potent regulation of cell mediated immunity and anti-arthritic activity.

The compound I has the structural formula:

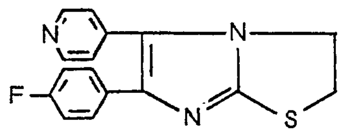

I

The compound I of this invention is usually used in its free base form however pharmaceutically acceptable acid addition salts or its oxide derivatives at the S atom may be used equivalently.

The pharmaceutically acceptable acid addition salts of the compound of Formula I are formed with strong or moderately strong organic or inorganic acids by methods known to the art. It will be appreciated that there is one basic center in the imidazole ring and another in the 4-pyridyl substituent.

For example, the base is reacted with an organic or inorganic acid in aqueous miscible solvent, such as ethanol or isopropanol, with isolation of the salt by removing the solvent or in an aqueous immiscible solvent when the acid is soluble therein, such as ethyl ether or chloroform, with the desired salt separating directly or being isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts. As stated above the base has physical properties which make its use equivalent to that of its salt derivatives.

The sulfoxide derivative, that is the compound of Formula I, in which the 1-sulfinyl group is present, is prepared by oxidation of the 2,3-dihydroimidazo[2,1-b]thiazole, preferably with one equivalent of *m*-chloroperbenzoic acid in a solvent such as methylene chloride or with a similar sulfur oxidizing agent such as sodium or potassium periodate, hydrogen peroxide or other organic peracids.

The sulfone compound, that is the compound of Formula I in which the 1-sulfonyl group is present, is obtained by oxidation of either the 2,3-dihydroimidazo[2,1-b]thiazole or its sulfoxide derivative with two or one equivalents of oxidizing agent, respectively, in a suitable organic solvent.

The compound I of this invention is prepared by alkylation of 4-(4-pyridyl)-5-(4-fluorophenyl)-2-mercaptoimidazole with an ethylene 1,2-halide having reactive halo atoms such as iodo, chloro or bromo, for example 1-bromo-2-chloroethane, 1,2-dibromoethane or 1-iodo-2-chloroethane in an organic solvent in which the reactants are soluble. With the use of acidic solvents the alkylation-cyclization proceeds to give the hydrohalide salt form of the compound of Formula I. Other polar solvents can also be used especially in the presence of an alkali metal reagent capable of forming the alkali metal derivative of the 2-mercaptan group of the starting material such as sodium or potassium hydrides or lower alkoxides. The solvent is dimethylformamide, dimethylacetamide, dimethylsulfoxide, acetonitrile, hexamethylphosphoramide.

The reaction is allowed to proceed to completion at temperatures from room temperature or with moderate heating such as reflux temperature. The acid addition salt product which is a mixture of isomers at the 5,6-position is separated into the individual isomers by methods known to the art for example fractional recrystallization or chromatography. Alternatively the salt is converted into the free base mixture which is then separated into the individual isomer, the desired 5-(4-pyridyl)-6-(4-fluorophenyl)-2,3-dihydroimidazo-[2,1-b]-thiazole.

From DE—A—2 709 639 and DE—A—2 742 725, a series of imidazo-[2,1-b]-thiazoles are known having two phenyl groups which can be substituted in different ways. In spite of the immuno regulating and antiarthritis activity of these prior art compounds, the properties of the claimed compounds were not foreseeable.

A European patent application, No. 353, was published on January 24, 1979 which discloses a broad group of compounds related to the species here claimed. The species whose structure is closest to that claimed here and which is exemplified in Example 6 of the European patent is the mixture of isomers, 5(6)-(3-pyridyl)-6(5)-phenylimidazo[2,1-b]dihydrothiazole. The key structural features of the species claimed here, the 4-fluorophenyl, the 4-pyridyl and the proper isomeric structure, are not disclosed in the European specification.

Activity useful in the treatment of arthritis is determined by the following test procedures:

Inhibition of adjuvant induced polyarthritis in rats, as measured by reduction of rat paw edema, is produced by the compound of this invention at daily doses of about 3.125—50 mg/kg orally with indomethacin and methotrexate controls. In this test procedure, adjuvant arthritis in rats is produced by a single intradermal injection of 0.75 mg of *Mycobacterium butyricum* suspended in white paraffin oil into the left hindpaw footpad. The injected paw becomes inflamed (increased volume) and reaches maximal size within three to five days (primary lesion). The animals exhibit a decrease in body weight

gain during the initial period. The adjuvant arthritis (secondary lesion) occurs after approximately ten days and is characterized by inflammation of the non-injected right hind leg, decrease in body weight, and further increase in the volume of the injected left hind leg. Test compounds are administered daily, beginning on the day of the adjuvant injection, for 17 days thereafter, exclusive of days 4, 5, 11 and 12. Anti-arthritic activity is shown by the ability to protect the animals against the development of both primary and secondary lesions of adjuvant arthritis.

In this test 5-(4-pyridyl)-6-(4-fluorophenyl)-2,3-dihydroimidazo[2,1-b]thiazole demonstrated positive activity at 6.25, 12.5, 25 and 50 mg/kg orally to give an oral $ED_{25}$ of 26.4 (15.6—54.4) mg/kg/day left leg volume day 3, 10.4 (5.3—19.0) mg/kg/day left leg volume day 16 and 7.5 (2.7—15.6) mg/kg/day right leg volume day 16. ($ED_{25}$ refers to the calculated dose which produces a 25% decrease from control values.)

On the contrary the transposed isomer, 6-(4-pyridyl)-5-(4-fluorophenyl-2,3-dihydroimidazo[2,1-b]thiazole demonstrated no activity at 50 mg/kg/day. Nor did a closely related analog 5,6-bis-(4-pyridyl)-2,3-dihydroimidazo[2,1-b]thiazole (50 mg/kg/day).

In the carrageenan induced rat paw edema test, anti-inflammatory activity is produced by the active compound of this invention at doses of 50 and 100 mg/kg orally.

In addition, compounds which have immunoregulatory activity provide benefit for treatment of rheumatoid arthritis. It has been found that the species of this invention demonstrate the ability to regulate cell-mediated immunity as shown in standard procedures such as the oxazolone-induced contact sensitivity test procedure in which mouse paw is measured. This procedure is described by Griswold et al., *Cellular Immunology* 11:198—204 (1974).

In the oxazolone sensitivity test 5-(4-pyridyl-6-(4-fluorophenyl)-2,3-dihydroimidazo[2,1-b]thiazole demonstrated significant activity after oral administration.

| Dose mg/kg | Difference in Edema Volume | % Change From Control | |
|---|---|---|---|
| 25 | 24.2 ± 7.8 | +69 | significant |
| control | 14.3 ± 2.4 | | |
| 10 | 25.3 ± 5.9 | +86 | significant |
| 1 | 27.2 ± 14.3 | +100 | significant |
| 0.1 | 18.5 ± 1.9 | +36 | not significant |
| control | 13.6 ± 3.8 | | |

The transposed isomer demonstrated no significant activity at 25 mg/kg orally.

| | | | |
|---|---|---|---|
| 25 | 16.0 ± 3.7 | +15 | not significant |
| control | 13.9 ± 3.0 | | |

Also the 5,6-di(2-pyridyl) analogue demonstrated no significant activity as 25 mg/kg orally.

| | | | |
|---|---|---|---|
| 25 | 15.3 ± 3.5 | −7 | not significant |
| control | 16.5 ± 3.8 | | |

The compound I of this invention had an oral $LD_{50}$ of 214.2 mg/kg orally in mice over 14 days.

In addition to having utility in rheumatoid arthritis, immunoregulatory agents have potential utility in other diseases where cell mediated immunity is compromised. Examples of such diseases are systemic lupus erythematosus and autoimmune thyroiditis. Also, diseases such as atopic dermatitis, recurrent aphthus ulceration, recurrent upper respiratory tract infections in children and flu, lung and breast cancer, transient granulocytopenia and allergic skin reactions have been successfully treated with levamisole which is an agent which restores impaired cell mediated immune response.

The pharmaceutically effective compounds of this invention are administered in conventional dosage unit forms prepared by combining the compound of Formula I in an amount sufficient to produce antiarthritic activity or regulation of cell mediated immunity with standard pharmaceutical carriers according to conventional procedures. These procedures may involve mixing, granulating and compressing or dissolving the ingredients as appropriate to the desired preparation.

The pharmaceutical carrier employed may be, for example, either a solid or liquid. Exemplary of solid carriers are lactose, terra alba, sucrose, talc, gelatin, agar, pectin, acacia, magnesium stearate,

stearic acid and the like. Exemplary of liquid carriers are syrup, peanut oil, olive oil, water and the like. Similarly, the carrier or diluent may include time delay material well known to the art, such as glyceryl monostrearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25 mg to about 1 g. If a liquid carrier is used, the preparation will be in the form of a syrup, emulsion, soft gelatin capsule, sterile injectable liquid such as an ampule or nonaqueous liquid suspension. The oral dosage unit forms are most useful.

To obtain a stable water soluble dose form, a pharmaceutically acceptable acid addition salt, preferably sulfate, of the compound of Formula I is dissolved in an aqueous solution of an organic or inorganic acid, such as a 0.3 M solution of succinic acid or, preferably, citric acid. In addition to sulfate, exemplary of other water soluble salts are methanesulfonate, phosphate and hydrochloride.

Preferably, each dosage unit will contain the active ingredient in an amount of from about 25 mg to about 100 mg.

The following examples are not limiting but are illustrative of the invention. Temperatures are on the Centigrade scale.

Example 1

To an aqueous solution (75 ml) of sodium cyanide (19.6 g; 400 mm) and benzyltriethyl ammonium chloride (3.0 g, 13 mm) was added isonicotinaldehyde (10 g, 93 mm) in 100 ml of methylene chloride at 0° temperature. Vigorous stirring was maintained for 15 minutes and a methylene chloride solution of benzoyl chloride (14.0 g, 100 mm, 50 ml) was slowly added. Cooling was discontinued after one-half hour reaction and the mixture was allowed to reach ambient temperature. The organic layer was separated and washed with 5% sodium carbonate and brine, dried and evaporated to an oil which was thoroughly extracted with ether (4 l). The ethereal solution was concentrated to approximately 500 ml and allowed to crystallize to give 5.0 g of isonicotinaldehyde-O-benzoylcyanohydrin. The cyanohydrin (3.0 g, 12 mm) was stirred in 50 ml of *tert.*-butyl alcohol with *p*-fluorobenzaldehyde (12 mm) and sodium hydride (12 mm) was added. Stirring was continued for 1—1/2 hours at room temperature. A mineral oil suspension of potassium hydride (24% suspension, 4 ml, 23 mm) was added with caution. A thin layer chromatographic assay of an aliquot of the reaction mixture after 1—1/2 hours indicated the formation of a single product. This was worked up by quenching the suspension in 200 ml of ice-water mixture then extracting the quench with chloroform. The chloroform extract was evaporated to a crystalline residue which was further crystallized by the addition of ether. A crystalline product was obtained which could optionally be purified by crystallization from methylene chloride-ether mixtures. This was not necessary for satisfactory yields.

9.0 g (37 mm) of the hydroxy ketone was refluxed in 140 ml of dimethylformamide with 5.3 g (70 mm) of thiourea. Starting material was no longer detected after 4 hours of reaction. The solvent was concentrated to one half of the original volume to initiate the precipitation of the crystalline 2-mercaptoimidazole; crystallization was completed overnight in the refrigerator. The compound was purified by crystallization from ethanol.

4-(4-Pyridyl)-5-(4-fluorophenyl)-2-mercaptoimidazole was formed in 40% overall yield of crude material based on the benzoyl cyanohydrin condensation, m.p. 386—388°.

Anal. Calcd. 1/8 $H_2O$: C, 61.46; H, 3.78; N, 15.39 Anal. Calcd. 1/8 HO:
Found: C, 61.58; H, 4.21; N, 15.11 Found:

The 2-mercaptoimidazole (12.5 mm) was suspended in 100 ml of dimethylformamide and sodium hydride (13.0 mm) was added. Salt formation was allowed to proceed at room temperature for 1/2 hour at which time 1-bromo-2-chloroethane was added from a syringe and the solution was stirred overnight under an argon atmosphere.

Solid anhydrous potassium carbonate (20.0 mm) was added to the reaction mixture and reflux was initiated for 2—1/2 hours. Dilution of the dimethylformamide solution with ice-water mixture to 300 ml, caused precipitation of an oily product.

The oily residue was chromatographed over silica using the dry column technique with 1:1 ethyl acetate/ether. This separated the isomeric mixture from all impurities. The isomers were then separated by medium pressure liquid chromatography on silica with isopropanol/ether (1:2). The faster moving spot ($R_f$:0.55, silica/isopropanol) after elution was further purified by crystallization from isopropanol, m.p. 165—167°, yield 0. 75 g of the 5-(4-fluorophenyl)-6-(4-pyridyl) isomer.

Anal. Calcd.: C, 64.63; H, 4.07; N, 14.13
Found: C, 64.85; H, 4.12; N, 14.12

The slow moving spot, the preferred 5-(4-pyridyl)-6-(4-fluorophenyl) isomer ($R_f$:0.25, silica/isopropanol) after elution was recrystallized from isopropanol: m.p. 186—189°, 0.62 g.

Anal. Calcd.: C, 64.63; H, 4.07; N, 14.13
Found: C, 64.27; H, 4.10; N, 14.05

This compound is converted to its pharmaceutically acceptable acid addition salts such as the dihydrochloride, sulfate, methane sulfonate and others by reaction of the base with an excess of acid in an organic solvent such as isopropanol.

Example 2

To 1.9 g of 6-(4-fluorophenyl)-5-(4-pyridyl)-2,3-dihydroimidazo[2,1-b]thiazole in 25 ml of methylene chloride cooled to ice-bath temperature was added 1.36 g of *m*-chloroperbenzoic acid. Stirring was continued for 1 hour then the solution was diluted with methylene chloride and washed with a 1:1 mixture of 5% sodium carbonate and brine. Treating with magnesium sulfate, filtering and stripping gave 2.1 g of 6-(4-fluorophenyl)-5-(4-pyridyl-2,3,-dihydroimidazo[2,1-b]thiazole-1-oxide which was triturated with ether, filtered and dried to give 1.8 g, m.p. 229—231°.

Anal. Calcd.: C, 61.33; H, 3.86; N, 13.41
Found: C, 61.61; H, 4.18; N, 13.46

Treating the starting material with 2.72 g of *m*-chloroperbenzoic acid gives the 1-dioxide derivative.

Example 3

To 50 ml glacial acetic acid was added 1.0 g of 4-(4-pyridyl)-5-(4-fluorophenyl)-2-mercaptoimidazole and 0.7 g of dibromoethane. The milky solution clarified after refluxing for one hour. At this point an additional 2.1 g dibromoethane was added and refluxing was continued for an additional 3 hours. The acetic acid was stripped off *in vacuo.* The residue was partitioned between methylene chloride and 5N sodium carbonate solution. The organic extract was washed with 5N sodium carbonate solution and brine then treated with magnesium sulfate. Evaporation of the solvent left a mixture of 6-(4-fluorophenyl)-5-(4-pyridyl)-2,3-dihydroimidazo[2,1-b]thiazole and 5-(4-fluorophenyl)-6-(4-pyridyl)-2,3-dihydro-imidazo[2,1-b]thiazole which was separated as described above.

Example 4

| Ingredients | Amounts |
|---|---|
| 5-(4-Pyridyl)-6-(4-fluorophenyl)-2,3-dihydroimidazo[2,1-b]thiazole | 100 mg |
| Magnesium stearate | 5 mg |
| Lactose | 50 mg |

The above ingredients are screened, mixed and filled into a hard gelatin capsule. The capsule is administered to a patient in need of treatment for arthritis or to regulate cell mediated immunity from 1—6 times daily orally.

**Claims for the Contracting States BE, CH, DE, FR, GB, IT, LU, NL, SE**

1. The compound of the structure:

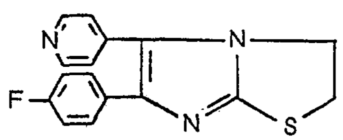

or a pharmaceutically acceptable acid addition salt or S-oxide derivatives thereof.

2. 5-(4-Pyridyl)-6-(4-fluorophenyl)-2,3-dihydroimidazo[2,1-b]thiazole base.

3. A medical composition having regulation of cell mediated immunity and antiarthritic activity comprising, as an active ingredient, a compound of claims 1 or 2 plus a pharmaceutical carrier.

4. The method of preparing 5-(4-pyridyl)-6-(4-fluorophenyl)-2,3-dihydroimidazo[2,1-b]thiazole comprising reacting 4-(4-pyridyl)-5-(4-fluorophenyl)-2-mercaptoimidazole with an ethylene dihalide said halide being chloro, bromo or iodo to obtain a mixture of 5-(4-pyridyl)-6-(4-fluorophenyl)-2,3-dihydroimidazo[2,1-b]thiazole and 5-(4-fluorophenyl)-6-(4-pyridyl)-2,3-dihydroimidazo[2,1-b]thiazole then separating the mixture by methods known to the art.

**Claim for the Contracting State AT**

The method of preparing 5-(4-pyridyl)-6-(4-fluorophenyl)-2,3-dihydroimidazo[2,1-b]thiazole and its pharmaceutically acceptable acid addition salts and sulfoxide and sulfone derivatives comprising reacting 4-(4-pyridyl)-5-(4-fluorophenyl)-2-mercaptoimidazole with an ethylene dihalide, said halide being chloro, bromo or iodo, to obtain a mixture of 5-(4-pyridyl)-6-(4-fluorophenyl)-2,3-dihydroimidazo[2,1-b]thiazole and 5-(4-fluorophenyl-6-(4-pyridyl)-2,3-dihydroimidazo[2,1-b]thiazole, then separating the mixture by methods known to the art to isolate 5-(4-pyridyl)-6-4-fluorophenyl)-2,3-dihydroimidazo[2,1-b]thiazole and optionally forming an acid addition salt thereof by reaction with a pharmaceutically acceptable acid or the S-oxide derivatives thereof by reaction with a sulfur oxidizing agent.

**Patentansprüche für die Vertragsstaaten BE, CH, DE, FR, GB, IT, LU, NL, SE**

1. Verbindung der Formel

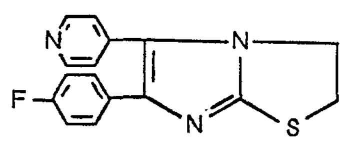

I

oder ihr pharmazeutisch verträgliches Säureadditionssalz oder ihre S-Oxid-Derivate.

2. 5-(4-Pyridyl)-6-(4-fluorphenyl)-2,3-dihydroimidazo [2,1-b]thiazolbase.

3. Arzneimittel mit regulierender Wirkung auf die zelluläre Immunität und mit antiarthritischer Wirkung enthaltend als aktiven Wirkstoff eine Verbindung der Ansprüche 1 oder 2 und einen pharmazeutischen Träger.

4. Verfahren zur Herstellung von 5-(4-Pyridyl)-6-(4-fluorphenyl)-2,3-dihydroimidazo[2,1-b]thiazol durch Umsetzung von 4-(4-Pyridyl)-5-(4-fluorphenyl)-2-mercaptoimidazol mit einem Äthylendihalogenid, wobei das Halogen ein Chlor, Brom- oder Jodatom bedeutet, zu einem Gemisch von 5-(4-Pyridyl)-6-(4-fluorphenyl)-2,3-dihydroimidazo[2,1-b]thiazol und 5-(4-Fluorphenyl)-6-(4-pyridyl)-2,3-dihydroimidazo[2,1-b]thiazol und anschließendem Trennen des Gemisches in an sich bekannter Weise.

**Patentanspruch für den Vertragsstaat AT**

Verfahren zur Herstellung von 5-(4-Pyridyl)-6-(4-fluorphenyl)-2,3-dihydroimidazo[2,1-b]thiazol und seiner pharmazeutisch verträglichen Säureadditionssalze und Sulfoxid- und Sulfonderivate, gekennzeichnet durch Umsetzung von 4-(4-Pyridyl)-5-(4-fluorphenyl)-2-mercaptoimidazol mit einem Äthylendihalogenid, wobei das Halogen ein Chlor-, Brom- oder Jodatom bedeutet, zu einem Gemisch aus 5-(4-Pyridyl)-6-(4-fluorphenyl)-2,3-dihydroimidazo[2,1-b]thiazol und 5-(4-Fluorphenyl)-6-(4-pyridyl)-2,3-dihydroimidazo[2,1-b]thiazol, und anschließendes Trennen des Gemisches auf an sich bekannte Weise zur Isolierung von 5-(4-Pyridyl)-6-(4-fluorphenyl)-2,3-dihydroimidazo[2,1-b]thiazol und gegebenenfalls Überführen mit einer pharmazeutisch verträglichen Säure in sein Säureadditionssalz oder durch Umsetzen mit einem schwefeloxidierenden Mittel in seine S-Oxidderivate.

**Revendications pour les Etats Contractants BE, CH, DE, FR, GB, IT, LU, NL, SE**

1. Composé de structure:

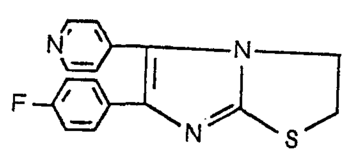

I

ou ses sels d'addition avec un acide pharmaceutiquement acceptable ou ses dérive5s S-oxydes.

2. La base 5-(4-pyridyl)-6-(4-fluorophényl)-2,3-dihydroimidazo[2,1-b]thiazole.

3. Composition médicale présentant un pouvoir de régulation de l'immunité à médiation cellulaire et une activité antiarthritique comprenant, à titre d'ingrédient actif, un composé des revendications 1 ou 2 plus un excipient pharmaceutique.

4. Procédé de préparation du 5-(4-pyridyl)-6-(4-fluorophényl)-2,3-dihydroimidazo[2,1-b]thiazole

qui consiste à faire réagir le 4-(4-pyridyl)-5-(4-fluorophényl)-2-mercaptoimidazole avec un dihalogénure d'éthylène, ledit halogénure étant un groupe chloro, bromo, ou iodo, pour obtenir un mélange de 5-(4-pyridyl)-6-(4-fluorophényl)-2,3-dihydroimidazo[2,1-b]thiazole et 5-(4-fluorophényl)-6-(4-pyridyl)-2,3-dihydroimidazo[2,-1-b]thiazole, et à séparer ensuite le mélange par des méthodes connues dans la technique.

**Revendication pour l'Etat contractant AT**

Procédé de préparation du 5-(4-pyridyl)-6-(4-fluorophényl)-2,3-dihydroimidazo[2,1-b]thiazole ainsi que de ses sels d'addition d'acide pharmaceutiquement acceptables et des dérivés sulfoxydes et sulfones qui consiste à faire réagir le 4-(4-pyridyl)-5-(4-fluorophényl)-2-mercaptoimidazole avec un dihalogénure d'éthylène, ledit halogénure étant un group chloro, bromo, ou iodo, pour obtenir un mélange de 5-(4-pyridyl)-6-(4-fluorophényl)-2,3-dihydroimidazo[2,1-b]thiazole et 5-(4-fluorophényl)-6-(4-pyridyl)-2,3-dihydroimidazo[2,1-b]thiazole, á séparer ensuite le mélange par des méthodes connues dans la technique pour isoler le 5-(4-pyridyl)-6-(4-fluorophényl)-2,3-dihydroimidazo[2,1-b]-thiazole, et facultativement à préparer un de ses sels d'addition d'acide par la réaction avec un acide pharmaceutiquement acceptable ou avec ses dérivés S-oxydes par la réaction avec un agent d'oxydation du soufre.